# EUROPÄISCHE PATENTSCHRIFT

(11) **EP 1 259 177 B1**
(45) Veröffentlichungstag und Bekanntmachung des Hinweises auf die Patenterteilung: **01.09.2004**
(21) Anmeldenummer: 00904794.5
(22) Anmeldetag: 29.02.2000
(51) Int. Cl.: A61B 17/66, A61C 8/00

(54) **ENDO-DISTRAKTOR**
ENDO-DISTRACTOR
ENDOSEPARATEUR

(43) Veröffentlichungstag der Anmeldung: 27.11.2002
(73) Patentinhaber: Synthes AG Chur, 7002 Chur (CH)
(72) Erfinder: KRENKEL, Christian, A-5020 Salzburg (AT); LIXL, Georg, A-5110 Oberndorf (AT)
(74) Vertreter: Lusuardi, Werther Giovanni, Dr.
(86) Internationale Anmeldenummer: PCT/CH2000/000117
(87) Internationale Veröffentlichungsnummer: WO 2001/064118

(56) Entgegenhaltungen:
- WO-A-99/37230
- DE-A- 19 822 802
- US-A- 5 961 329

## Beschreibung

Die Erfindung bezieht sich auf eine Vorrichtung zur Distraktion von Knochen oder Knochensegmenten gemäss dem Oberbegriff des Patentanspruchs 1.

Zur gezielten Verschiebung von Knochenfragmenten relativ zueinander werden in der heutigen chirurgischen Operationstechnik externe oder auch interne Distraktoren eingesetzt. Speziell bei der Kieferchirurgie eignen sich Endo-Distraktoren. Dies vor allem wegen der geringen Störungen für den Patienten während des oft Wochen dauernden Distraktionsvorganges.

Ein Endo-Distraktor geeignet für Kieferchirurgie ist beispielweise aus der internationalen Patentanmeldung WO 99 37230 A bekannt.

Am zahnlosen Unterkiefer gelangen Distraktoren auch dort zum Einsatz, wo sich der Unterkiefer stark zurückgebildet hat. Neben der kosmetischen Problematik kann auch die Implantation des künstlichen Zahnersatzes kritisch sein. Für die Zahnersatzimplantate fehlt die nötige Knochensubstanz, was zu einer Auslockerung der Implantate oder gar zum Bruch des Unterkiefers führen kann. Die krankhafte Rückbildung des Unterkiefers ist nicht nur beim alten Patienten vorhanden, sondern kann auch schon beim 40-jährigen Patienten vorkommen, was den Entscheid zu einem operativen Eingriff vereinfacht.

Um den Unterkiefer wieder aufzubauen, wird der Unterkiefer in horizontaler Richtung osteotomiert. Danach wird der Osteotomiespalt kontinuierlich vergrössert. Diese Distraktion das Osteotomiespaltes geschieht mit ca. ½ mm pro Tag. Währen dem langsamen Distraktionsvorgang bildet sich Knochengewebe im Ostoetomiespalt. Nach erfolgter Distraktion muss die Distanz des Osteotomiespaltes über eine gewisse Zeit gehalten werden.

Der Erfindung liegt daher die Aufgabe zugrunde, einen. Endo-Distraktor zu schaffen, welcher den Patienten in seiner Bewegungsfreiheit nur geringfügig einschränkt.

Die Erfindung löst die gestellte Aufgabe mit einer Vorrichtung zur Distraktion von Knochen oder Knochensegmenten, welche die Merkmale des Anspruchs 1 aufweist.

Die erfindungsgemässe Vorrichtung zur Distraktion von Knochen oder Knochensegmenten umfasst eine Verankerungsschraube, welche eine Längsachse, einen Gewindeschaft, einen kreiszylindrischen Schraubenschaft und ein Schraubenende mit Mitteln zum rotativen Antrieb der Verankerungsschraube aufweist, und eine auf dem Schraubenschaft axial verschiebbare Distraktionshülse, welche koaxial zur Längsachse hohlzylindrisch ausgebildet ist, ein hinteres Ende, ein vorderes Ende, eine kreiszylindrische Durchgangsbohrung, an das vordere Ende angrenzend ein Aussengewinde und am hinteren Ende Kupplungsmittel für ein Eindrehwerkzeug aufweist. Ferner enthält die Verankerungsschraube zwischen Gewindeschaft und Schraubenschaft einen radial mindestens über den Schraubenschaft vorstehenden Bund, welcher als axialer Anschlag für das vordere Ende der über den Schraubenschaft geschobenen Distraktionshülse dient. Die Durchgangsbohrung umfasst an das hintere Ende angrenzend einen Bohrungsabschnitt mit erweitertem Querschnitt, worin koaxial ein Dichtungsring zwischen der Mantelfläche des Bohrungsabschnittes und dem Schraubenschaft angeordnet ist. Das Aussengewinde auf der Distraktionshülse ist vorzugsweise als Linksgewinde ausgeführt.

In der bevorzugten Ausführungsform der erfindungsgemässen Vorrichtung weist der Bund einen in den Schraubenschaft mündenden Konus auf. Damit wird die Kontaktfläche zwischen dem Konus und dem vorderen Ende der Distraktionshülse minimiert, womit der Vorteil erzielbar ist, dass die Verankerungsschraube trotz axialer Druckbelastung zwischen Verankerungsschraube und Distraktionshülse leicht um die Längsachse rotierbar bleibt. Anstelle des konischen Übergangs ist auch ein sphärisch konvexer Übergang möglich.

Je nach Dichtungstyp kann der Übergang zwischen dem Bohrungsabschnitt mit erweitertem Querschnitt und der Durchgangsbohrung in der Distraktionshülse als Planfläche oder als Innenkonus, welcher in die Durchgangsbohrung mündet, ausgestaltet sein. Damit lässt sich eine geeignete axiale Auflagefläche für den Dichtungsring erzielen.

Die erfindungsgemässe Vorrichtung umfasst zusätzlich eine Abdeckschraube, welche in eine am Schraubenende angebrachte Bohrung mit Innengewinde eindrehbar ist. Die Abdeckschraube bildet den mechanischen Abschluss des Distraktors in der Mundhöhle und sichert zudem die Verankerungsschraube gegenüber der Distraktionshülse.

In einer weiteren Ausführungsform der erfindungsgemässen Vorrichtung ist das Schraubengewinde selbstschneidend ausgebildet, wobei eine oder mehrere Schneidnuten angebracht sein können. Der Aussendurchmesser des Schraubengewindes liegt in einem Bereich zwischen 2,2 mm bis 3,5 mm, beträgt jedoch vorzugsweise 2,7 mm. Das Gewindeprofil ist asymmetrisch ausgebildet, wobei vorzugsweise die gegen die Schraubenspitze gerichteten Gewindeflanken mit der Längsachse einen Winkel zwischen 80° und 90° einschliessen. Damit wird die Druckbelastung im wesentlichen axial auf den weichen Knochen übertragen. Der Durchmesser des Schraubenschaftes liegt in einem Bereich zwischen 1,8 mm bis 2,5 mm, beträgt jedoch vorzugsweise 2,2 mm. Die Mittel zum Antrieb der Verankerungsschraube dürfen den Schraubenschaft radial nicht überragen und bestehen beispielsweise aus polygonartig angeordneten und gleichmässig auf dem Umfang des Schraubenschaftes verteilten Flächen und bilden vorzugsweise einen Vierkant oder Sechskant.

Ebenfalls vorteilhaft ist die Ausgestaltung des Aussengewindes an der Distraktionshülse mit einem asymmetrisches Gewindeprofil, wobei die gegen das hintere Ende gerichteten Gewindeflanken mit der Längsachse einen Winkel zwischen 80° und 90° einschliessen. Auch hier lässt sich somit eine im wesentlichen axiale Übertragung der Druckbelastung auf den Knochen erreichen.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung:
- Die Distraktion im Zentrum des Schwerpunktes der Distraktion erfolgt und das Distraktionsgerät dadurch nicht auf Biegung belastet wird sondern nur auf axialen Druck. Dadurch lässt sich das erfindungsgemässe Endo-Distraktionsgerät mit kleinen Abmessungen gestalten;
- Die Distraktion nach der Osteotomie horizontal versetzt beginnen kann und deshalb gleichzeitig eine horizontale Verschiebeosteotomie ist, wobei die Versetzung gezielt einsetzbar ist;
- Die Distraktion nach der Osteotomie in verschiedenen Richtungen erfolgen kann, je nachdem wie der Winkel zur Osteotomieebene für die Bohrungen für den Endo-Distraktor gewählt wird. Es lassen sich dadurch eine schlechte Relation des Ober- und Unterkieferkammes für die spätere prothetische Versorgung verbessern;
- Die Distraktionshülse mit ihrer Abdeckschraube in der monatelangen Retentionszeit zur Stabilisierung einer provisorischen Prothese dienen und ähnlich wie ein Zahnimplantat wirken kann;
- Die Frühbelastung des Endo-Distraktors und damit des frisch distrahierten Knochens mit seinen funktionellen Reizen zu einer Beschleunigung der Knochenbildung im Distraktionsgebiet führt;
- Das Gewinde der Distraktionshülse gegenläufig und somit gegen Ausdrehen während der Distraktion gesichert ist;
- Das Linksgewinde der Distraktionshülse nach deren Entfernung ein knöchernes Linksgewinde hinterlässst. Dieses Gewinde mit dem Rechtsgewinde im unteren Knochensegment sind bereits die Pilotbohrung für das Implantat, das in der selben Operation nach der Entfernung des Endo-Distraktors gesetzt wird. Diese Gewindelöcher sind daher gleichzeitig die Pilotbohrungen für das nachgesetzte Zahnimplantat;
- Das knöcherne Linksgewinde des Hohlimplantates nach dem Schneiden eines Rechtsgewindes für das Zahnimplantat ein sicheres neues Knochengewinde mit einem guten Primären Sitz des Zahnimplantates ergibt. Die beiden gegenläufigen Gewinde kreuzen sich unterschiedlich je nach Unterschied der Höhe der Gewindegänge; und
- Aus dem vorher genannten Vorteil alle Typen von Zahnimplantaten mit den unterschiedlichsten Gewinden zum Einsatz kommen können und der Chirurg nicht auf eine Type eines Implantates für die prothetische Versorgung festgelegt wird.

Mit Hilfe der erfindungsgemässen Vorrichtung lässt sich eine Unterkieferdistraktion mit der folgenden Operationstechnik durchführen:

Der Unterkiefer wird über spezielle, weichteilschonende Zugänge horizontal gespalten (Osteotomie). Je nach Situation werden zwei oder mehrere erfindungsgemässe Endo-Distraktoren angewendet. Die Verankerungsschrauben der Endo-Distraktoren werden durch das kraniale Knochensegment, durch den Osteotomiespalt, in das kaudale Knochensegment eingebracht. Die Gewindespitzen der Verankerungsschrauben werden über das kaudale Knochensegement hinaus eingedreht. Die Überlänge entspricht der zu erzielenden Distraktion im Frakturspalt. Die Distraktionshülsen werden in das kraniale Knochensegment eingesetzt und blockieren die Verankerungsschrauben in kranialer Richtung.

Der eigentliche Distraktionsvorgang erfolgt nun über mehrere Tage oder Wochen und wird meist vom Patienten selbst durchgeführt. Der Patient dreht dabei die Verankerungsschraube um ca. ½ mm pro Tag retour. Da die Distraktionshülse das Herausdrehen der Verankerungsschraube in kranialer Richtung verhindert, wird das kraniale Knochensegment mit Hilfe der Distraktionshülse nach kranial angehoben.

Der Vorteil dieser Operationstechnik ist der minimale technische Aufwand auf der Seite der Implantate und deren geringes Volumen.

Speziell ist eine vertikale Distraktion der Alveolarfortsätze des Ober- und Unterkiefers nach dem Salzburger Konzept ausführbar. Ganz anders verhält es sich, wenn vor der Insertion der Implantate der Alveolarfortsatz wieder auf seine ursprünglich Höhe "aufgebaut" werden kann:
- Der Abstand zwischen den zahnlosen Alveolarfortsätzen im Oberkiefer und Unterkiefer entsprechen wieder der Höhe von natürlichen Zähnen und die Implantataufbauten sind niedrig und damit stabil;
- Es bestehen keine schwer zu reinigenden Nischen unter den Implantaten; und
- Der Unterkiefer ist nach der Distraktion wieder mächtig in Höhe und Breite, lange und damit stabile Implantate können gesetzt werden, ohne der Gefahr einer Spontanfraktur.

Gegenüber anderen Hohlimplantaten weist das erfindungsgemässe Endo-Distraktions-Implantat folgende Unterschiede auf:
1. Das Gegenlager (mit axialer und radialer Funktion) für die Verankerungsschraube ist in einer Distraktionshülse im äusseren Knochensegment eingebaut, die innen kein Gewinde benötigt und während der gesamten Distraktions- und Retentionszeit mit Hilfe von O-Ringen die Verankerungsschraube bakterien- und speicheldicht abgeschlossen hält.
2. Die Verankerungsschraube wird nicht mehr mittels eines Metallgewindes geführt, sondern in einem eigens im natürlichen Knochen der Gegenseite des Osteotomiespaltes geschnittenen Gewindebohrung.
3. Die sichere Richtungsführung der Distraktion ist durch die Gewindebohrung im kortikalen Knochen gewährleistet.
4. Die Distraktion erfolgt immer im Zentrum des Knochens.
5. Die Osteotomielinie ist aussen an keiner Stelle durch Metall abgedeckt, dies ist eine Idealbedingung für die Kallusbildung.
6. Die Reservelänge der Verankerungsschraube für das Ausmass der Distraktion ist immer gegenüber der Seite der Instrumentenbedienung tief in den Weichteilen versteckt und führt daher zu keiner Beeinträchtigung für den Patienten.
7. Die erfindungsgemässe, als Endodistraktions-Implantat ausgebildete Vorrichtung kann in 2 grundsätzlichen Anwendungsarten mit jeweils 2 verschiedenen untereinander kombinierbaren Typen zum Einsatz kommen:

Anwendungsart a): Als Distraktionsgerät, bei dem das distal von Haut oder Schleimhaut gelegene Osteotomiefragment, das das Knochengewinde für die Verankerungsschraube führt, den ortsstabilen Teil, das haut- oder schleimhautnahe Osteotomiefragment mit der Distraktionshülse den beweglichen Teil darstellt, der bei der Distraktionsbehandlung vom stabilen Hauptfragment abgehoben wird. Einsatz z.B. bei breitflächiger Alveolarfortsatzdistraktion im Ober- und Unterkiefer, regionaler Alveolarkammdistraktion, Jochbeindistraktion, Unterkiefer-Collum-Distraktion, sowie in anderen Knochenregionen des Körpers, z. B.: Fingerdistraktion.
Anwendungsart b): Als Distraktionsgerät, bei dem das distal von Haut oder Schleimhaut gelegene Osteotomiefragment, das das Knochengewinde für die Verankerungsschraube führt, den beweglichen Teil, das haut- oder schleimhautnahe Osteotomiefragment mit der Distraktionshülse den ortsstabilen Teil darstellt, der während der Distraktionsbehandlung, den beweglichen Knochenteil mit dem Knochengewinde von sich wegschiebt. Einsatz z.B. bei Kinn-Augmentationsdistraktion, Unterkieferrand-Augmentationsdistraktion, Bone-Transport bei Defekten im Unterkieferast im Zusammenspiel mit der funktionsdynamischen Überbrückungsplatte sowie in anderen Knochenregionen des Körpers.
c) Typ 1: Anwendung der als Endodistraktions-Implantat ausgebildeten erfindungsgemässen Vorrichtung, bei dem die Distraktionshülse mit Anschlag für die Verankerungsschraube als enossal eingeschraubtes Implantat mit dem Osteotomiesegment in festem Verbund steht. Einsatz z. B. bei breitflächiger Alveolarfortsatzdistraktion im Ober- und Unterkiefer, regionaler Alveolarfortsatzdistraktion.
d) Typ 2: Anwendung der als Endodistraktions-Implantat ausgebildeten erfindungsgemässen Vorrichtung, bei dem das Gegenlager mit Anschlag für die Verankerungsschraube mittels Miniplättchen gerade oder in unterschiedlichen Winkeln zum Knochen je nach Erfordernis mit Plättchenschrauben stabilisiert wird. Einsatz z. B. bei Jochbeindistraktion, Kinn-Augmentationsdistraktion; Unterkieferrand-Augmentationsdistraktion, Unterkiefer-Collum-Distraktion, Bone-Transport bei Defekten im Unterkieferast im Zusammenspiel mit der funktionsdynamischen Überbrückungsplatte.

Anwendungsgebiete für das erfindungsgemässe Endo-Distraktions-Implantat sind:
1. breitflächige Alveolarfortsatzdistraktion im Unterkiefer;
2. breitflächige Alveolarfortsatzdistraktion im Oberkiefer;
3. regionale Alveolarkammdistraktion;
4. Jochbeindistraktion;
5. Kinn-Augmentationsdistraktion;
6. Unterkieferrand-Augmentationsdistraktion;
7. Unterkiefer-Collum-Distraktion;
8. Bone-Transport bei Defekten im Unterkieferast im Zusammenspiel mit der funktionsdynamischen Überbrückungsplatte; und
9. in anderen Knochenregionen des Körpers, z. B. Fingerdistraktion

Als Implantatsysteme für die prothetischen Aufbauten kommen hauptsächlich in Frage:
- Ankylos Implantate
- Bonefit Implantate
- Frialit II Implantate
- Bränemark Implantate

Die Distraktionshülse der erfindungsgemässen, als Endodistrakor ausgeführten Vorrichtung hat mehrere Funktionen:
1. Widerlager, um das obere osteotomierte Segment anzuheben;
2. Zahnfleischformer für das später zu setzende Implantat;
3. Geschützter Zugang zur Innenschraube, um diese drehen zu können;
4. Abdichtungsmöglichkeit mit Knetwachs durch eine versenkte Innenschraube. Ein Niveauunterschied von 0,5 mm zwischen dem Oberrand der Distraktionshülse und dem Ende der Verankerungsschraube erscheint ausreichend, um die Knetwachskügelchen sicher aufzunehmen; und
5. Aufnahmebuchse für O-Ringe, die einen speichel- und bakteriendichten Abschluss zum Knochen garantieren.

Die Abdeckschraube schliesst den Totraum der Distraktionshülse ab, hat die Funktion wie ein Gingivaformer und bietet einen gewissen Halt für eine provlsorische Prothese.

Die Implantation wird vorzugsweise nach folgender Methode durchgeführt:
. Operationsanleitung der Mandibuladistraktion im anterioren Bereich;
. Anästhesie;
. Vorbereitungen;
. Operationszeichnung;
. Schnittführung;
. Vermessung;
. Planung der horizontalen Osteotomie;
. Horizontale Osteotomie;
. Bohrungen für das Implantat;
. Gewindeschneiden:
   2 x Linksgewinde schneiden (∅ 4,0 mm) im oberen Segment:
      Wiederum mit Hilfe der Ratsche werden unter guter Kühlung die Gewinde für bis alle Knochenspäne aus den Knochengewinden entfernt sind.
. Eindrehen der Implantate:
   Eindrehen der linksgewindigen Hohlimplantate mit O-Ringdichtung:
      Die beiden Distraktionshülsen werden mit dem entsprechenden Kronenschlüssel und der Ratsche in die beiden Gewinde des oberen Segmentes bis auf Anschlag eingedreht. Dabei ist darauf zu achten, dass die beiden O-Ringe im Inneren der Distraktinshülse nicht verloren gehen, da sie die Garantie darstellen, dass Speichel, Nahrungsreste und Keime nicht bis zum Knochen vordringen können.
. Sicherung des Implantates;
. Osteoplastik;
. Wundverschluss;
. Frühe postoperative Zeit;
. Distraktionszeit;
. Retentionszeit; und
. Setzen der Zahnimplantate.

Ist die Ossifikation gut erfolgt, können die Zahnimplantate gesetzt werden.

Wichtig ist es, dass die längsten Implantate verwendet werden, die zur Verfügung stehen, denn nur lange Implantate können auch das kaudale Osteotomiefragment erreichen und auf diese Weise die Distraktion überbrücken, was einer Schrumpfungstendenz des neugebildeten Knochens entgegenwirkt. Zur Entfernung der erfindungsgemässen Endodistraktions-Implantate wird ein einfacher Kieferkammschnitt durchgeführt und die Implantatregion direkt dargestellt. Zirkelangaben vom Setzen der Implantate sind hilfreich für das neuerliche Auffinden der Lokalisation für die seitlichen Implantate in sicherem Abstand vom Nervus mentalis. Die äusseren Implantate werden in der üblichen Weise zuerst gesetzt. Bohrspäne beim Setzen dieser Implantate werden sorgfältig mit dem Knochensauger gewonnen und in Zellnährlösung zwischengelagert. Dann werden mit dem Kronenschlüssel und der Ratsche die Distraktionshülsen im Uhrzeigersinn (Linksgewinde) ausgedreht, danach die Verankerungsschrauben mit ihrem speziellen Schlüssel, dies jedoch im Gegenuhrzeigersinn (Rechtsgewinde). Nun erfolgt die Knochenpräparation für die beiden mesialen Implantate, wobei jeder Knochen, der gewonnen werden kann, sorgfältig in Zellnährlösung zwischengelagert wird. Ergeben sich zwischen den gesetzten Implantaten und dem Knochen in dieser Region Dehiszenzen, werden diese sorgfältig kürettiert und mit Knochenspänen und Knochenmehl aufgefüllt. Eine semipermeable Membran über dem Implantat verhindert die Resorption des Knochens und das Einwachsen von Bindegewebe. Nach Mobilisation der Schleimhäute werden diese doppelschichtig mit langsam resorbierbaren Fäden geschlossen. Die Nahtentfernung erfolgt 14 Tage nach der Implantation. Ein Prothesenprovisorium kann ab einer Woche nach Setzen der Implantate angefertigt werden. Es ist zu beachten, dass die Zentrik des Bisses genau stimmt und die Prothesen in kurzen Abständen immer wieder weich unterfüttert werden.

Der wichtigste Vorteil der erfindungsgemässen Vorrichtung besteht darin, dass im Mundvorhof nicht ein zweites Mal operiert werden muss, um Implantatteile zu entfernen. Ferner können die alten Bohrungen für das definitive Zahnimplantat genützt werden und dies kann in örtlicher Anästhesie einfach und dem Patienten leicht zumutbar durchgeführt werden.

Weitere Vorteile sind:
- Der Arbeitsteil des Endodistraktions-Implantates befindet sich relativ zur Haut- oder Schleimhautoberfläche immer in der gleichen Höhe, so dass eine baldige Anfertigung von Prothesenprovisorien möglich ist;
- Die Implantatteile können von mindestens drei auf nur zwei reduziert werden;
- Die Distraktion kann gezielt auch in der Osteotomieebene versetzt gestartet werden;
- Durch den Einsatz verschiedener Bohrungswinkel kann die Distraktion in unterschiedlichen Neigungswinkeln je nach Erfordernis erfolgen;
- Eine restlose, einfache Implantatentfernung ist allein durch das Ausdrehen der Distraktionshülse und der Verankerungsschraube möglich;
- Nach der Implantatentfernung kann sofort mit allen gängigen Implantatsystemen das definitive Zahnimplantat gesetzt werden, was einen zusätzlichen operativen Eingriff erspart;
- Das auf das absolute Minimum reduzierte Distraktionssystem ist einfach in der Anwendung und mit sehr wenigen Instrumenten ausführbar;
- Das Distraktionssystem ist für die verschiedensten Anwendungsgebiete immer gleich, deshalb in der Handhabung leicht erlernbar und universell mit den immer gleichen Teilen anwendbar;
- Beim Einsetzen werden keine Teile mechanisch verformt, so dass es nicht vorkommen kann, dass ein Implantat schon vor der Anwendung bei den Adaptionsarbeiten beschädigt werden kann; und
- Ein und dasselbe Distraktionsgerät kann nach Misslingen einer Applikation während einer Operation an anderer Lokalisationen neu gesetzt werden, das Gerät durch die erste Applikation keine Bearbeitungsspuren zeigt, das heisst unbeschädigt ist.

Werden zwei erfindungsgemässe Distraktionsvorrichtungen zur Verdrehungssicherung und Aufteilung der Distraktionskräfte benötigt, so ergeben sich dafür zwei verschiedene Möglichkeiten:
a) Die beiden Endodistraktions-Implantate werden in weitem Abstand parallel voneinander gesetzt: Die tägliche seitengleiche Distraktion wird mit einem Schlüssel an beiden Geräten durch genau gleiches Drehen nacheinander erreicht (eine Markierung am Schlüssel ist erforderlich).
b) Zwei Geräte werden eng zueinander gesetzt und mit einem Ansatzgerät bedient, das simultan beide Gewindestangen um den gleichen Drehwinkel in derselben Drehrichtung rotiert und damit eine parallele Distraktion erreicht. Ein solches Ansatzgerät arbeitet mit drei in Serie ineinandergreifenden Zahnrädern, von denen das mittlere mit seiner Achse in Verbindung zum Drehschlüssel steht und die beiden äusseren mit ihren Achsen die Gewindestangen antreiben.

Zudem lassen sich mit der erfindungsgemässen Vorrichtung die weiteren Vorteile erzielen:
- Das Linksgewinde des Hohlimplantates gibt eine zusätzliche Sicherheit bei der Bedienung des Rechtsgewindes des Gewindestange während der Distraktion. Bei einer eventuellen leichten Lockerung desHohlimplantates im Knochengewinde des oberen Osteotomiesegmentes wird dieses durch das Drehmoment der Gewindestange im Hohlimplantat beim heraus drehen wieder fest gedreht;
- Wesentliche Verbesserung, da die Bauteile der bisherigen Geräte von drei auf zwei grundsätzlich notwendige Bauteile verringert werden konnten;
- Ferner ist ein und dasselbe Gerät universell für verschiedenste Indikationen anwendbar;
- Erspart die Operation der Entfernung des Gerätes, da diese im selben Operationsschritt mit dem Setzen der Zahnimplantate zusammenfällt und schon die Bohrungen schafft, die für die Zahnimplantate benötigt werden;
- Speichel- und bakteriendichter Abschluss in der Region der Knochendistraktion;
- Es werden keine unnatürlich geformten Teile innerhalb der Mundhöhle aber auch außerhalb in der Gesichtsregion benötigt, so dass keine Entstellung entsteht oder der Patient beim Essen, Sprechen oder bei der Mundhygiene gestört wird;
- Der erfindungsgemässe Endo-Distraktor weist die äussere Form eines Einzelzahnimplantates auf und ist geeignet, wie ein Zahn oder Zahnimplantat in der Retentionszeit schon einen Halt zu geben; und
- Der erfindungsgemässe Endo-Distraktor ist klein, jedoch bezüglich seiner Distraktionslänge leistungsfähig, arbeitet im Knochenzentrum in der statisch günstigsten Region und die Bauteile sind nur axial auf Zug und Druck belastet (keine seitlichen Scher- und Biegekräfte vieler bekannter anderer Geräte), wodurch die größtmögliche Sicherheit gegen Materialermüdungsbrüche garantiert wird.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch die bevorzugte Ausführungsform der erfindungsgemässen Vorrichtung;
Fig. 2 einen Schnitt durch einen Unterkiefer zu Beginn des Distraktionsvorganges mit einer eingesetzten erfindungsgemässen Vorrichtung;
Fig. 3 einen Schnitt durch einen Unterkiefer während des Distraktionsvorganges mit einer eingesetzten erfindungsgemässen Vorrichtung; und
Fig. 4 einen Schnitt durch einen Unterkiefer während des Distraktionsvorganges mit zwei eingesetzten erfindungsgemässen Vorrichtungen.

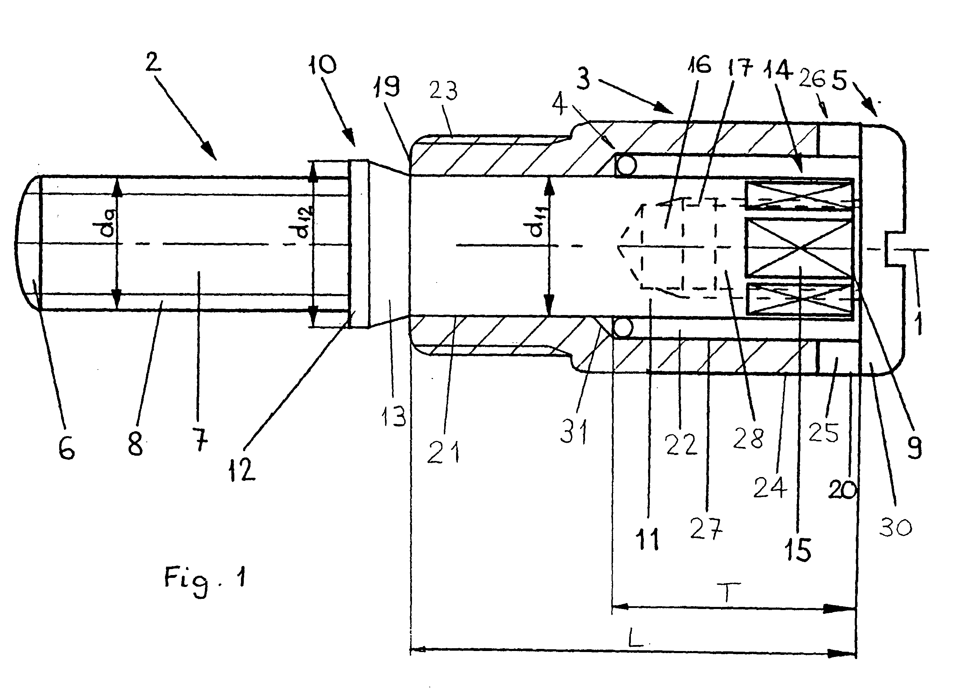

In Fig. 1 ist die bevorzugte Ausführungsform der erfindungsgemässen Vorrichtung dargestellt. Diese umfasst eine Verankerungsschraube 2 mit einer Längsachse 1, sowie koaxial zur Längsachse 1 eine Distraktionshülse 3, einen Dichtungsring 4 und eine Abdeckschraube 5. Die Verankerungsschraube 2 enthält an eine abgerundete Schraubenspitze 6 anschliessend einen Gewindeschaft 7 mit einem Schraubengewinde 8, welcher sich ungefähr über die halbe Länge der Verankerungsschraube 2 erstreckt und einen Aussendurchmesser dₐ aufweist. Daran anschliessend und axial in Richtung des Schraubenendes 9 aufeinanderfolgend sind ein Bund 10 und ein kreiszylindrischer Schraubenschaft 11 mit dem Durchmesser d₁₁ angeordnet. Das Schraubengewinde 8 ist asymmetrisch mit einem Winkel von 87° zwischen den gegen die Schraubenspitze 6 gerichteten Gewindeflanken und der Zentralachse 1 ausgestaltet. Der Bund 10 weist gegen den Gewindeschaft 7 gerichtet einen kreiszylindrischen Abschnitt 12 mit einem Durchmesser d₁₂ auf, wobei der Durchmesser d₁₂ grösser als der Aussendurchmesser dₐ und grösser als der Durchmesser d₁₁ des Schraubenschaftes 11 ist. Gegen den Schraubenschaft 11 gerichtet umfasst der Bund 10 einen Konus 13, welcher vom Abschnitt 12 sich verjüngend in den Schraubenschaft 11 mündet. An des Schraubenende 9 angrenzend sind auf dem Schraubenschaft 11 die Mittel 14 zum Antrieb der Verankerungsschraube 2 angebracht, welche hier als Sechskant mit sechs auf dem Umfang des Schraubenschaftes 11 gleichmässig angeordneten Flächen 15 ausgeführt sind. Am Schraubenende 9 ist eine zur Längsachse 1 koaxiale Bohrung 16 mit einem Innengewinde 17 angebracht.

Die Distraktionshülse 3 ist koaxial zur Längsachse 1 hohlzylindrisch ausgestaltet und weist ein vorderes Ende 19, ein hinteres Ende 20 und parallel zur Längsachse 1 eine Länge L auf. An das vordere Ende 19 angrenzend umfasst die Distraktionshülse 3 ein Aussengewinde 23, welches sich über die Hälfte der Länge L erstreckt, während das an das hintere Ende 20 angrenzende Hülsensegment 24 im Querschnitt erweitert ist und am hinteren Ende 20 vier zueinander orthogonale Schlitze 25 als Kupplungsmittel 26 für ein Eindrehwerkzeug aufweist. Die koaxial zur Längsachse 1 verlaufende Durchgangsbohrung 21 ist kreiszylindrisch ausgestaltet und weist einen Durchmesser auf, welcher eine axiale Verschiebbarkeit der Distraktionshülse 3 auf dem Schraubenschaft 11 ermöglicht. Vom hinteren Ende 20 her ist die Durchgangsbohrung 21 auf einem Bohrungsabschnitt 22 bis zu einer Tiefe T im Querschnitt erweitert, wobei der Bohrungsabschnitt 22 konisch mit einem halben Konuswinkel von 60° in die Durchgangsbohrung 21 mündet und die Tiefe T so bemessen ist, dass sich der Bohrungsabschnitt 22 nicht über das gesamte Hülsensegment 24 erstreckt. Die Distraktionshülse 3 lässt sich vom Schraubenende 9 her über den Schraubenschaft 11 schieben bis das vordere Ende 19 am Konus 13 anschlägt, wobei die Distraktionshülse 3 auf dem Schraubenschaft 11 um die Längsachse 1 frei rotierbar bleibt und das hintere Ende 20 über das Schraubenende 9 vorsteht.

Zwischen der Mantelfläche 27 des Bohrungsabschnittes 22 und dem Schraubenschaft 11 ist ein Dichtungsring 4 als Sperre zwischen der Mundhöhle und dem Kiefer eingebracht.

Zudem umfasst die Vorrichtung eine Abdeckschraube 5, deren Gewindeteil 28 in das Innengewinde 17 am Schraubenende 9 eindrehbar ist bis der Schraubenkopf 30 am hinteren Ende 20 der Distraktionshülse 3 ansteht.

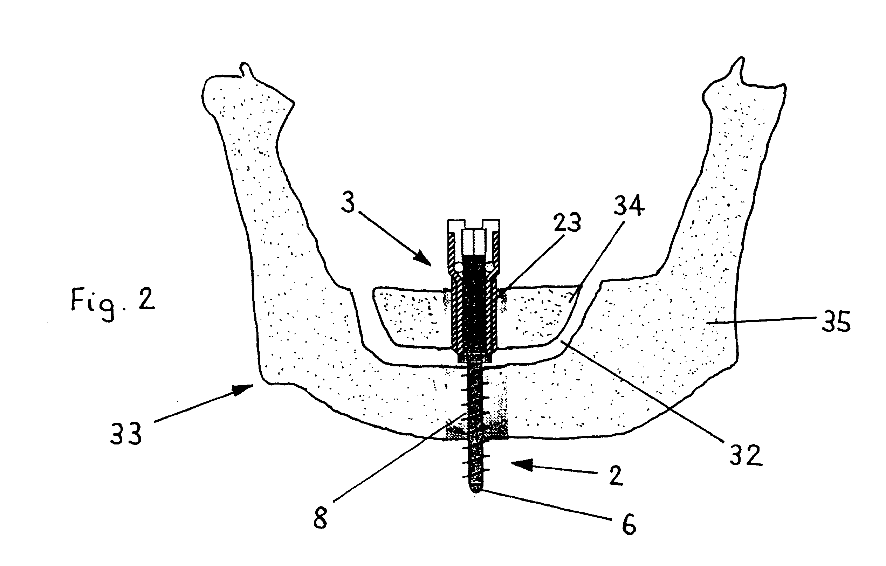

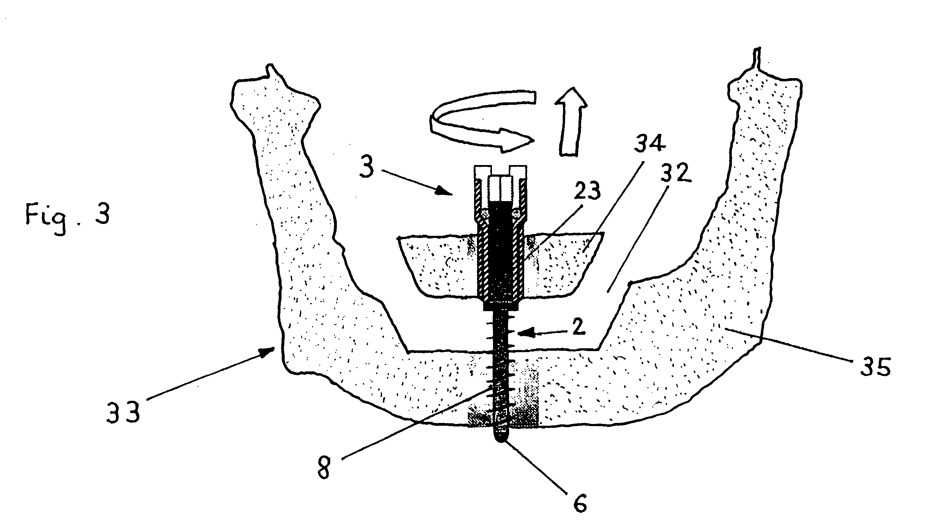

In den Fig. 2 und 3 ist ein Distraktionsvorgang am Beispiel eines osteotomierten Unterkieferknochens 33 mit dem Osteotomiespalt 32 dargestellt. Dabei handelt es sich um einen Distraktionsvorgang gemäss Anwendungsart a), bei welchem der Alveolarfortsatz nach kranial verschoben wird. Wie oben beschrieben werden die Verankerungsschrauben 2 der Endo-Distraktoren durch das kraniale Knochensegment 34 und durch den Osteotomiespalt 32, in das kaudale Knochensegment 35 eingebracht. Die Gewindespitzen 6 der Verankerungsschrauben 2 werden über das kaudale Knochensegement 35 hinaus eingedreht. Die Überlänge entspricht der zu erzielenden Distraktion im Frakturspalt. Die Distraktionshülsen 3 werden in das kraniale Knochensegment 34 eingesetzt und blockieren die Verankerungsschrauben 2 in kranialer Richtung. Beim Retourdrehen der Verankerungsschraube 2 wird das kraniale Knochensegment 34 mit Hilfe der Distraktionshülse 3 kranial angehoben, wobei das kaudale Knochensegment 35 ortsstabil bleibt.

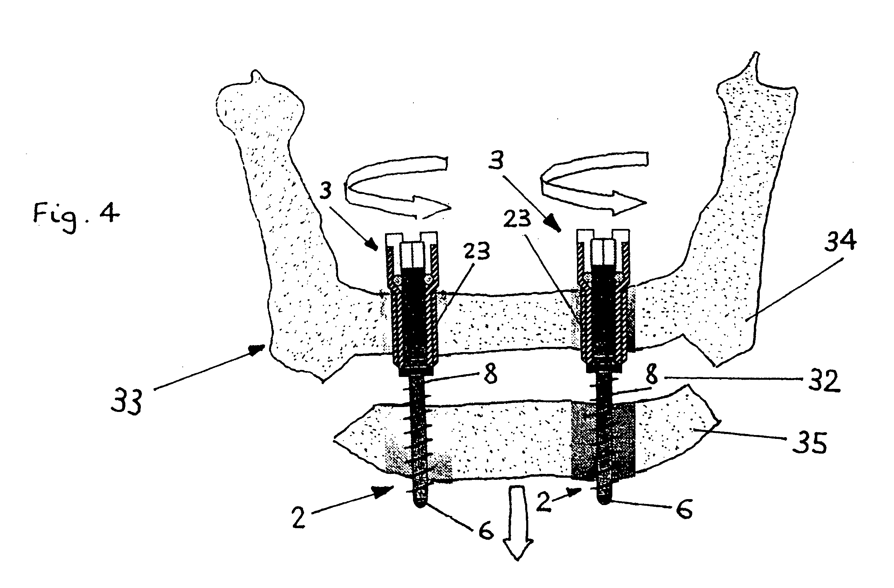

In Fig. 4 wird der Distraktionsvorgang gemäss Anwendungsart b) ebenfalls am Beispiel eines osteotomierten Unterkieferknochens 33 illustriert. Hier erfolgt bei der Distraktion eine Verschiebung des Kinns nach kaudal.

## Patentansprüche

1. Vorrichtung zur Distraktion von Knochen oder Knochensegmenten mit
A) einer Verankerungsschraube (2), welche eine Längsachse (1), eine Schraubenspitze (6), ein Schraubenende (9) mit Mitteln (14) zum rotativen Antrieb der Verankerungsschraube (2), einen an die Schraubenspitze (6) grenzenden Gewindeschaft (7) mit einem Schraubengewinde (8) und einen an das Schraubenende (6) grenzenden kreiszylindrischen und gewindelosen Schraubenschaft (11) umfasst; und
B) einer auf dem Schraubenschaft (11) axial verschiebbaren Distraktionshülse (3), welche ein hinteres Ende (20), ein vorderes Ende (19), eine zwischen dem hinteren Ende (20) und dem vorderen Ende (19) koaxial verlaufende Durchgangsbohrung (21), an das vordere Ende (19) angrenzend ein Aussengewinde (23) und am hinteren Ende (20) Kupplungsmittel (26) für ein Eindrehwerkzeug aufweist,
**dadurch gekennzeichnet, dass**
C) die Verankerungsschraube (2) zwischen Gewindeschaft (7) und Schraubenschaft (11) einen radial mindestens über den Schraubenschaft (11) vorstehenden Bund (10) umfasst, welcher als axialer Anschlag für das vordere Ende (19) der über den Schraubenschaft (11) geschobenen Distraktionshülse (3) dient;
D) die Durchgangsbohrung (21) auf einem an das hintere Ende (20) angrenzenden und bis zu einer Tiefe (T) reichenden Bohrungsabschnitt (22) im Querschnitt erweitert ist und auf dem Bohrungsabschnitt (22) eine Mantelfläche (27) aufweist; und
E) ein Dichtungsring (4) koaxial zwischen Mantelfläche (27) und Schraubenschaft (11) angeordnet ist.

2. Vorrichtung nach Anspruch 1, **dadurch gekennzeichnet, dass** der Bund (10) einen Konus (13) umfasst, welcher in den Schraubenschaft (11) mündet.

3. Vorrichtung nach Anspruch 1, **dadurch gekennzeichnet, dass** der Bund (10) eine sphärisch konvexe Verjüngung umfasst, welche in den Schraubenschaft (11) mündet.

4. Vorrichtung nach einem der Ansprüche 1 bis 3, **dadurch gekennzeichnet, dass** der Bohrungsabschnitt (22) mit einem Innenkonus (31) in die Durchgangsbohrung (21) mündet.

5. Vorrichtung nach einem der Ansprüche 1 bis 4, **dadurch gekennzeichnet, dass** sie zusätzliche eine Abdeckschraube (5) umfasst und am Schraubenende (9) eine Bohrung (16) mit einem Innengewinde (17) angebracht ist, worin die Abdeckschraube (5) eindrehbar ist.

6. Vorrichtung nach einem der Ansprüche 1 bis 5, **dadurch gekennzeichnet, dass** das Schraubengewinde (8) selbstschneidend ist.

7. Vorrichtung nach einem der Ansprüche 1 bis 6, **dadurch gekennzeichnet, dass** das Schraubengewinde (8) ein asymmetrisches Gewindeprofil aufweist.

8. Vorrichtung nach Anspruch 7, **dadurch gekennzeichnet, dass** die gegen die Schraubenspitze (6) gerichteten Gewindeflanken mit der Längsachse (1) einen Winkel zwischen 80° und 90° einschliessen.

9. Vorrichtung nach einem der Ansprüche 1 bis 8, **dadurch gekennzeichnet, dass** das Aussengewinde (23) ein asymmetrisches Gewindeprofil aufweist.

10. Vorrichtung nach Anspruch 9, **dadurch gekennzeichnet, dass** die gegen das hintere Ende (20) gerichteten Gewindeflanken mit der Längsachse (1) einen Winkel zwischen 80° und 90° einschliessen.

11. Vorrichtung nach einem der Ansprüche 1 bis 10, **dadurch gekennzeichnet, dass** das Gewindeprofil des Aussengewindes (23) abgeflachte Gewindespitzen aufweist.

12. Vorrichtung nach einem der Ansprüche 1 bis 11, **dadurch gekennzeichnet, dass** die Mittel (14) radial nicht über den Schraubenschaft (11) vorstehen.

## Claims

1. A device for the distraction of bones or bone segments including
A) an anchoring screw (2) which comprises a longitudinal axis (1), a screw tip (6), a screw end (9) with means (14) for rotatably driving the anchoring screw (2), a threaded shank (7) which adjoins the screw tip (6) and has a screw thread (8), and a circular cylindrical and non-threaded screw shank (11) adjoining the screw end (9); and
B) a distraction sleeve (3) axially displaceable on the screw shank (11) which has a rear end (20), a front end (19), a continuous boring (21) extending coaxially between the rear end (20) and the front end (19), an external screw thread (23) adjoining the front end (19), and coupling means (26) formed in the rear end (20) for receiving a screwing-in tool,
**characterised in that**
C) the anchoring screw (2) comprises a collar (10) arranged between the threaded shank (7) and the screw shank (11) which radially projects at least from the screw shank (11) and which serves as an axial stop for the front end (19) of the distraction sleeve (3) that is slid over the screw shank (11);
D) the continuous boring (21) has a boring section (22) with an enlarged cross-section which adjoins the rear end (20) and extends to a depth (T), said boring section (22) being provided with a lateral surface (27); and
E) a seal ring (4) is coaxially arranged between the lateral surface (27) and the screw shank (11).

2. A device as claimed in claim 1, **characterised in that** the collar (10) comprises a cone (13) which leads to the screw shank (11).

3. A device as claimed in claim 1, **characterised in that** the collar (10) comprises a spherically convex, tapered portion which leads to the screw shank (11).

4. A device as claimed in any of the claims 1 to 3, **characterised in that** the boring section (22) has an inner cone (31) which leads to the continuous boring (21).

5. A device as claimed in any of the claims 1 to 4, **characterised in that** it comprises in addition a cover screw (5) and that the screw end (9) is provided with a bore (16) having an internal screw thread (17) into which said cover screw (5) may be screwed.

6. A device as claimed in any of the claims 1 to 5, **characterised in that** the screw thread (8) is self-tapping.

7. A device as claimed in any of the claims 1 to 6, **characterised in that** the screw thread (8) has an asymmetrical thread profile.

8. A device as claimed in claim 7, **characterised in that** the flanks of the screw thread directed towards the screw tip (6) and the longitudinal axis (1) form an angle of between 80 and 90 degrees.

9. A device as claimed in any of the claims 1 to 8, **characterised in that** the external screw thread (23) has an asymmetrical thread profile.

10. A device as claimed in claim 9, **characterised in that** the flanks of the screw thread directed towards the rear end (20) and the longitudinal axis (1) form an angle of between 80 and 90 degrees.

11. A device as claimed in any of the claims 1 to 10, **characterised in that** the thread profile of the external screw thread (23) displays flattened, threaded tips.

12. A device as claimed in any of the claims 1 to 11, **characterised in that** the means (14) do not radially protrude from the screw shank (11).

## Revendications

1. Dispositif de distraction pour les os ou segments d'os, avec
A) une vis d'ancrage (2) contenant un axe longitudinal (1), une pointe de vis (6), une extrémité de vis (9) munie d'éléments (14) destinés à l'entraînement rotatif de la vis d'ancrage (2), une tige filetée (7) confinant à la pointe de vie (6) et munie d'un filet de vis (8) et une tige de vis (11) cylindrique et circulaire non filetée confinant à l'extrémité de vis (9); et
B) un manchon de distraction (3) mobile dans le sens axial sur la tige de vis (11 ) et contenant une extrémité arrière (20), une extrémité avant (19), un alésage traversant (21) dans le sens coaxial entre l'extrémité arrière (20) et l'extrémité avant (19), un filetage mâle (23) confinant à l'extrémité avant (19) et, sur l'extrémité arrière (20), des éléments d'accouplement (26) pour un outil de vissage,
**caractérisé en ce que**
C) la vis d'ancrage (2) contient, entre la tige filetée (7) et la tige de vis (11), une embase (10) dépassant radialement au moins de la tige de vis (11) et servant de butée axiale pour l'extrémité avant (19) du manchon de distraction (3) enfilé sur la tige de vis (11);
D) l'alésage traversant (21) contient une section élargie sur une partie de l'alésage (22) confinant à l'extrémité arrière (20) et atteignant une profondeur (T) et présente une surface de gaine (27) sur la partie d'alésage (22); et **en ce que**
E) une bague d'étanchéité (4) est disposée coaxialement entre la surface de gaine (27) et la tige de vis (11).

2. Dispositif selon la revendication 1, **caractérisé en ce que** l'embase (10) comprend un cône (13) qui débouche dans la tige de vis (11).

3. Dispositif selon la revendication 1, **caractérisé en ce que** l'embase (10) comprend un rétrécissement sphérique convexe qui débouche dans la tige de vis (11).

4. Dispositif selon une des revendications 1 à 3, **caractérisé en ce que** la partie d'alésage (22) débouche dans l'alésage traversant (21) au moyen d'un cône intérieur (31).

5. Dispositif selon une des revendications 1 à 4, **caractérisé en ce qu'**il présente en plus une vis de couverture (5) et **en ce que** l'extrémité de vis (9) est munie d'un alésage (16) à filetage femelle (17), dans lequel on peut introduire la vis de couverture (5).

6. Dispositif selon une des revendications 1 à 5, **caractérisé en ce que** le filet de vis (8) est autotaraudeur.

7. Dispositif selon une des revendications 1 à 6, **caractérisé en ce que** le filet de vis (8) présente un profil de filetage asymétrique.

8. Dispositif selon la revendication 7, **caractérisé en ce que** les flancs de filet orientés vers la pointe de vis (6) forment un angle de 80° à 90° avec l'axe longitudinal (1).

9. Dispositif selon une des revendications 1 à 8, **caractérisé en ce que** le filetage mâle (23) présente un profil de filetage asymétrique.

10. Dispositif selon la revendication 9, **caractérisé en ce que** les flancs de filet orientés vers l'extrémité arrière (20) forment un angle de 80° à 90° avec l'axe longitudinal (1).

11. Dispositif selon une des revendications 1 à 10, **caractérisé en ce que** le profil de filetage du filetage mâle (23) présente des sommets de filet aplatis.

12. Dispositif selon une des revendications 1 à 11, **caractérisé en ce que** les éléments (14) ne dépassent pas radialement de la tige de vis (11).
